(19) Europäisches Patentamt
European Patent Office
Office européen des brevets

(11) **EP 3 849 507 B1**

(12) **EUROPEAN PATENT SPECIFICATION**

(45) Date of publication and mention
of the grant of the patent:
**15.01.2025 Bulletin 2025/03**

(21) Application number: **19745150.3**

(22) Date of filing: **30.07.2019**

(51) International Patent Classification (IPC):
*A61K 8/37* *(2006.01)*   *A61K 8/55* *(2006.01)*
*A61K 8/58* *(2006.01)*   *A61Q 11/00* *(2006.01)*
*A61K 8/19* *(2006.01)*

(52) Cooperative Patent Classification (CPC):
**A61K 8/19; A61K 8/37; A61K 8/55; A61K 8/585;**
**A61Q 11/00;** A61K 2800/31

(86) International application number:
**PCT/EP2019/070454**

(87) International publication number:
**WO 2020/052855 (19.03.2020 Gazette 2020/12)**

(54) **ORAL CARE COMPOSITION**

MUNDPFLEGEZUSAMMENSETZUNG

COMPOSITION D'HYGIÈNE BUCCALE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **12.09.2018 EP 18193996**

(43) Date of publication of application:
**21.07.2021 Bulletin 2021/29**

(73) Proprietors:
• **UNILEVER GLOBAL IP LIMITED**
**Wirral**
**Merseyside CH62 4ZD (GB)**
Designated Contracting States:
**CY DE GB IE IT MT RS TR**
• **Unilever IP Holdings B.V.**
**3013 AL Rotterdam (NL)**
Designated Contracting States:
**AL AT BE BG CH CZ DK EE ES FI FR GR HR HU IS**
**LI LT LU LV MC MK NL NO PL PT RO SE SI SK SM**

(72) Inventors:
• **LIMER, Adam, John**
**Wirral Merseyside CH63 3JW (GB)**
• **LITTLEWOOD, David, Thomas**
**Wirral Merseyside CH63 3JW (GB)**

(74) Representative: **Tansley, Sally Elizabeth**
**Unilever Patent Group**
**Bronland 14**
**6708 WH Wageningen (NL)**

(56) References cited:
EP-B1- 2 906 182      WO-A1-2018/106219
KR-A- 20100 041 357   US-A- 3 937 803

## Description

### Field of the Invention

The present invention is concerned with oral care compositions. In particular the present invention is concerned with the mitigation of toothpaste compositions gassing.

### Background of the Invention

Toothpaste compositions can become unstable and have a formation of gas, causing tubes to swell and burst.
WO18106219 discloses "off-gas" reducing compositions comprising gas reducing components selected from selected from sodium sulphate, magnesium sulphate, citric acid and clay.

### Description of the Invention

The present invention relates to a non-aqueous personal care composition having a pH at 20°C of greater than 9.5 and comprising

    i) less than 1 wt% of the total composition of water;
    ii) and 0.1 to 10 wt% of the total composition of a calcium source comprising calcium carbonate.

The invention further relates to the use of a pH of greater than 9.5 to prevent gas formation in a toothpaste.

### Detailed Description of the Invention

The pH of the composition at 20°C is greater than 9.5. It is preferred if the composition has a pH at 20°C of less them 11, preferably less than 10.5.
The composition comprises a source of carbonate being calcium carbonate composed of primary particles.
By "primary particles" is meant individual particles, defined as the smallest discrete particles that can be seen by electron microscopy analysis (such as, for example, individual crystals).
The primary particles may associate under certain conditions to form larger secondary structures such as aggregates or agglomerates.
In one present invention, a suitable source of particulate calcium carbonate as defined above includes crystalline calcium carbonates in which the individual crystals have a prismatic morphology and an average size of 2 microns or less.
The term "crystalline" (in the context of particulate calcium carbonate) generally means a particulate calcium carbonate in which at least 50% by weight, preferably at least 75% by weight, more preferably at least 90% by weight, most preferably more than 95% by weight and ideally more than 99% by weight of the calcium carbonate particles are in the form of crystals.
The term "crystal" means an essentially fully dense solid composed of atoms arranged in an orderly repetitive array bounded by plane surfaces which are the external expression of internal structure. Crystals may be identified and characterised by standard techniques known to those skilled in the art such as X-ray diffraction.
Crystalline forms of calcium carbonate are available naturally, or may be synthetically produced in three particular crystalline morphologies, calcite, aragonite, and less commonly found, vaterite. The vaterite form of calcium carbonate is metastable and irreversibly transforms into calcite and aragonite. There are many different polymorphs (crystal habits) for each of these crystalline forms. The calcite crystalline morphology is the most commonly used crystal form of calcium carbonate. Over 300 crystalline forms of calcite have been reported in the literature.
References to a "prismatic crystal morphology" (in the context of crystalline calcium carbonates) generally denote a crystalline calcium carbonate in which at least 50% by weight, preferably at least 75% by weight, more preferably at least 90% by weight, most preferably more than 95% by weight and ideally more than 99% by weight of the crystals are prismatic crystals. Prismatic crystals have a set of faces with parallel edges that are parallel to the vertical or "c"-axis direction. There can be three, four, six, eight or even twelve faces that can form a prism. For the purposes of the present invention, preferred prismatic crystals have a generally uniform, typically generally hexagonal cross-sectional shape.
References to a "scalenohedral crystal morphology" (in the context of crystalline calcium carbonates) generally denote a crystalline calcium carbonate in which at least 50% by weight, preferably at least 75% by weight, more preferably at least 90% by weight, most preferably more than 95% by weight and ideally more than 99% by weight of the crystals are scalenohedral crystals. Scalenohedral crystals have a set of faces that are inclined to the vertical or "c"-axis direction, each of which are scalene triangles (i.e. triangles whose three sides are unequal in length). For the purposes of the present invention, preferred scalenohedral crystals have a trigonal scalenohedral shape, with twelve scalene triangle faces.

Reference to acicular" in this context refers to the shape of the crystals. Usually, crystals grow in three directions, length, width and height. Some crystals however, have one or two preferred growth directions. Acicular crystals have a preferred crystal growth in one direction. Examples are crystals in the form of needles, rods, fibres, whiskers or columns and the like. For the purposes of the present invention, preferred acicular crystals are rod-like or needle-like with a generally uniform, typically generally circular, cross-sectional shape.

Crystal shape may be determined by standard techniques known to those skilled in the art such as scanning electron microscopy (SEM). SEM is an imaging and analysis technique based on the detection of electrons and X-rays that are emitted from a material when irradiated by a scanning electron beam. Imaging allows the user to distinguish between primary particles and aggregates or agglomerates.

Usually, crystals grow in three directions, length, width and height. Some crystals however, have one or two preferred growth directions. For the purposes of the present invention, preferred prismatic calcium carbonate crystals have a ratio between the length and the width of the crystal (the so-called aspect ratio) ranging from about 1:1 up to about 3:1 (length:width). The aspect ratio of a particle (e.g. a crystal) may typically be obtained from SEM photographs by averaging the ratio between the length and width of the particle, measured on at least 10 particles for 1 photograph.

Preferably the calcium carbonate for use in this invention has a prismatic, scalenohedral or acicular crystal morphology.

For the purposes of the present invention, preferred prismatic calcium carbonate crystals have an average size which is less than 2 microns and generally ranges from about 0.1 to 2 microns, with a preferred size ranging from about 0.2 to about 1.5, more preferably from about 0.3 to about 1, most preferably from about 0.5 to 0.9 microns. Particle (e.g. crystal) size may be determined by standard techniques known to those skilled in the art such as sedimentation. Particle size values obtained from sedimentation techniques are normally expressed in terms of the equivalent spherical diameter (ESD), i.e. the diameter of a notional sphere having the same volume as the particle. The ESD value may be calculated based on the sedimentation rate of the particle in question as defined by Stokes' Law (Micromeritics SediGraph® 5100 Particle Size Analysis System Operator's Manual, V2.03, 1990). The sedimentation rate is measured using a finely collimated beam of low energy X-rays which pass through the sample cell to a detector. For a sample population of particles, the distribution of particle mass at various points in the cell affects the number of X-ray pulses reaching the detector. This X-ray pulse count is used to derive the particle size distribution expressed as the percent mass at given particle diameters. The median value of the ESD which can be derived from this distribution (i.e. the particular ESD value on the cumulative mass distribution curve at which 50 percent mass of the population has a higher ESD and 50 percent mass of the population has a lower ESD) is usually quoted as the average particle size of the sample population.

For the purposes of the present invention, preferred scalenohedral calcium carbonate crystals have an average size which is less than 2 microns and generally ranges from about 0.05 to about 1.5 microns, with a preferred size ranging from about 0.1 to about 1 micron, more preferably from about 0.15 to about 0.5 micron, most preferably from about 0.2 to 0.35 micron. A standard technique known to those skilled in the art for determining the average size of the individual crystals is air permeametry according to the Lea and Nurse method (standards NF X 11-601 and NF X 11 602). This analytical technique determines the average particle size by measuring the pressure drop across a packed powder bed using a water manometer. The pressure drop is a function of the permeability of the packed bed. This is related to the surface area of the particles which is then transformed to an average size based upon the assumption that the particles are spherical. Average particle size (Dp) is obtained from the massic area (SM) derived from the Lea and Nurse method by making the assumptions that all the particles are spherical, non-porous and of equal diameter, and by neglecting contact surfaces between the particles. The relationship between Dp and SM is the following:

$$Dp=6/(\rho SM)$$

where $\rho$ is the specific mass of the calcium carbonate. The average particle size (Dp) obtained in this way represents the average equivalent spherical diameter, or average ESD, where equivalent spherical diameter (ESD) is defined as the diameter of a notional sphere having the same volume as the particle.

For the purposes of the invention preferred acicular calcium carbonate is composed of a length of 2 microns or greater. Preferred acicular calcium carbonate crystals for use in the invention have a length ranging from 2 to 100 microns, more preferably from 10 to 30 microns and a width ranging from 0.1 to 4.0 microns, more preferably from 0.5 to 1.0 microns. A specific class of material suitable for use in the invention includes aragonite crystal form calcium carbonates such as those described for example in US 5,164,172.

The ratio between the length and the width of a crystal, the so-called aspect ratio, is higher than 1:1 for preferred acicular crystals. The higher the aspect ratio, the longer the crystal. For the purposes of the present invention the aspect ratio is preferably at least 2.5:1, more preferably at least 10:1. For example the aspect ratio may typically range from 2.5:1 to 200:1, and preferably ranges from 10:1 to 60:1, more preferably from 20:1 to 30:1.

In a preferred embodiment calcium carbonate has a particulate size less than 2 microns.

Preferred calcium carbonate crystals, in particular scalenohedral prismatic calcium carbonate generally have a BET specific surface area higher than or equal to 0.1 m2/g, preferably higher than or equal to 1 m2/g, more preferably higher than or equal to 3 m2/g and most preferably higher than or equal to 4 m2/g. The calcium carbonate particles according to the invention generally have a BET specific surface area lower than or equal to 30 m2/g, preferably lower than or equal to 25 m2/g, more preferably lower than or equal to 20 m2/g, and most preferably lower than or equal to 15 m2/g. The BET specific surface area is usually measured according to the standard ISO 9277 which specifies the determination of the overall specific external and internal surface area of disperse or porous solids by measuring the amount of physically adsorbed gas according to the Brunauer, Emmett and Teller (BET) method.

Crystalline forms of calcium carbonate suitable for use in the invention are available naturally, or may be produced by precipitation production technology. Typically, precipitated calcium carbonate is prepared by exposing calcium hydroxide slurry to a carbonation reaction. Control of the specific solution environment during the nucleation and growth of calcium carbonate governs the size and the shape of the crystals in the resulting precipitated calcium carbonate product.

A commercially available source of particulate prismatic calcium carbonate suitable for use in the invention is ViCALity® ALBAFIL® Precipitated Calcium Carbonate (PCC), ex Specialty Minerals Inc., Bethlehem, Pa. 18017.

A commercially available source of particulate scalenohedral calcium carbonate suitable for use in the invention is SOCAL® S2E Precipitated Calcium Carbonate (PCC), ex Solvay S.A.

Suitable acicular crystal form calcium carbonates for use in the invention are commercially available and include those marketed by Maruo Calcium Company Limited, Japan under the trade name WISCAL®.

Mixtures of any of the above described materials may also be used.

The total level of calcium or sodium carbonate, preferably calcium carbonate in the personal care composition is from 0.1 to 10.0 wt% of the total composition. More preferably from 0.5 to 7 wt% of the total composition.

The composition is preferably anhydrous in that the composition comprises less than 1wt% of the composition of water; preferably less than 0.5 wt%, more preferably less than 0.1 wt% of the total composition.

The composition preferably comprises a phosphate source. The phosphate source that may be used in this invention is limited only to the extent that the same may be used in a composition suitable for use in an oral cavity. Illustrative examples of the types of phosphate source suitable for use in this invention include monosodium phosphate, sodium dihydrogen phosphate, disodium hydrogen phosphate, sodium pyrophosphate, tetrasodium pyrophosphate, sodium tripolyphosphate, sodium hexametaphosphate, potassium dihydrogenphosphate, trisodium phosphate, tripotassium phosphate, mixtures thereof or the like. The phosphate source is preferably one which is water soluble.

Typically, the phosphate source makes up from 0.5 to 15%, and preferably, from 1 to 12%, and most preferably, from 2 to 9% by weight of the composition, based on total weight of the activator composition in which it is included and including all ranges subsumed therein. In a preferred embodiment, the phosphate source used is one which results in an oral care composition having a pH from 5.5 to 8, preferably from 6 to 7.5, and most preferably, about neutral. In a most preferred embodiment, the phosphate source used is trisodium phosphate and monosodium dihydrogen phosphate at a trisodium phosphate to monosodium dihydrogen phosphate weight ratio of 1:4 to 4:1, preferably 1:3 to 3:1, and most preferably, from 1:2 to 2:1, including all ratios subsumed therein.

It is preferable that the composition comprises a calcium source that is insoluble or slightly soluble in water.

Soluble means a source that dissolves in water to give a solution with a concentration of at least 0.1 moles per litre at room temperature. Insoluble means a source that dissolves in water to give a solution with a concentration of less than 0.001 moles per litre at room temperature. Slightly soluble, therefore, is defined to mean a source that dissolves in water to give a solution with a concentration of greater than 0.001 moles per litre at room temperature and less than 0.1 moles per litre at room temperature. Substantially free of, as used herein, means less than 1.5%, and preferably, less than 1.0%, and most preferably, from 0.0 to 0.75% by weight, based on total weight of the oral care composition, including all ranges subsumed therein. The calcium source suitable for use in this invention is limited only to the extent that the same may be used in an oral cavity. In a preferred embodiment, the calcium source employed is insoluble or slightly soluble in water, but most preferably, insoluble in water.

Illustrative examples of the types of calcium source that may be used in this invention include, for example, calcium phosphate (i.e., added), calcium gluconate, calcium oxide, calcium lactate, calcium carbonate, calcium hydroxide, calcium sulfate, calcium carboxymethyl cellulose, calcium alginate, calcium salts of citric acid, calcium silicate, calcium glycerophosphate and mixtures thereof. In a preferred embodiment, the calcium source is calcium silicate. In a more preferred embodiment, the calcium silicate used is ($CaS_iO_3$) whereby the same is made commercially available under the tradenames Sorbosil CA40 or PQ.

In yet another preferred embodiment, the calcium source is insoluble calcium silicate, present as the composite material calcium oxide-silica ($CaO$-$SiO_2$) as described in commonly-owned application Publication No. 2008/015117.

When a calcium silicate composite material is employed, the ratio of calcium to silicon (Ca:Si) may be from 1:10 to 3:1. The Ca:Si ratio is preferably from 1:5 to 2:1, and more preferably, from 1:3 to 2:1, and most preferably, from about 1:2 to 2:1. The calcium silicate may comprise mono-calcium silicate, bi-calcium silicate, or tri-calcium silicate whereby ratios of calcium to silicon (Ca:Si) should be understood to be atom ratios.

The calcium source employed in this invention may be in a crystalline or amorphous state, and preferably, the same is in an amorphous state. In an often-preferred embodiment, the calcium source is in a mesoporous state, i.e. the source is a material having pores with diameters from 1 nm to 50 microns. Mesoporous calcium silicate (MCS) is often preferred.

The MCS which may be used in this invention can be made by combining a calcium salt, a silica precursor like silicate and a structure-directing agent to yield a solid suitable for calcinating. A more detailed description of the process that may be conducted to make the MCS suitable for use in this invention is described in the aforementioned commonly-owned application, Publication No. WO 2008/015117.

Preferably the amount of calcium silicate in the composition is typically from 0.1 to 50%, and preferably, from 1 to 30%, and most preferably, from 5 to 20% by weight of the oral care composition based on total weight of the oral care composition.

A preferred form of calcium silicate is calcium silicate coated $TiO_2$. Examples of preferred forms of calcium silicate coated $TiO_2$ are disclosed in WO2012/031786 and WO2012/031785.

Preferably the composition is an oral care composition, more preferably a toothpaste.

The oral care composition described herein may comprise ingredients which are common in the art, such as:

- antimicrobial agents, e.g. Triclosan, chlorhexidine, copper-, zinc- and stannous salts such as zinc citrate, zinc sulphate, zinc glycinate, sodium zinc citrate and stannous pyrophosphate, sanguinarine extract, metronidazole, quaternary ammonium compounds, such as cetylpyridinium chloride; bis-guanides, such as chlorhexidine digluconate, hexetidine, octenidine, alexidine; and halogenated bisphenolic compounds such as 2,2' methylenebis-(4-chloro-6-bromophenol);
- anti-inflammatory agents such as ibuprofen, flurbiprofen, aspirin, indomethacin, etc.;
- anti-caries agents such as sodium trimetaphosphate and casein;
- plaque buffers such as urea, calcium lactate, calcium glycerophosphate and polyacrylates;
- vitamins such as Vitamins A, C and E;
- plant extracts;
- desensitizing agents, e.g. potassium citrate, potassium chloride, potassium tartrate, potassium bicarbonate, potassium oxalate, and potassium nitrate;
- anti-calculus agents, e.g. alkali-metal pyrophosphates, hypophosphite-containing polymers, organic phosphonates and phosphocitrates, etc;
- biomolecules, e.g. bacteriocins, antibodies, enzymes, etc;
- flavors, e.g., peppermint and spearmint oils;
- proteinaceous materials such as collagen;
- preservatives;
- opacifying agents;
- coloring agents like FD&C blue, yellow and/or red dyes/colorants;
- pH-adjusting agents;
- sweetening agents;
- surfactants, such as anionic, cationic and zwitterionic or amphoteric surfactants (e.g., sodium lauryl sulfate, sodium dodecylbenzene sulfonate);
- particulate abrasive materials such as abrasive silicas, aluminas, calcium carbonates, zirconium silicate, polymethylmethacrylate, dicalciumphosphates, calcium pyrophosphates, hydroxyapatites, trimetaphosphates, insoluble hexametaphosphates as well as agglomerated particulate abrasive materials;
- fluoride sources like sodium fluoride, stannous fluoride, sodium monofluorophosphate, zinc ammonium fluoride, tin ammonium fluoride, calcium fluoride, cobalt ammonium fluoride or mixtures thereof;
- polymeric compounds which can enhance the delivery of active ingredients such as antimicrobial agents can also be included. Examples of such polymers are copolymers of polyvinylmethylether with maleic anhydride and other similar delivery enhancing polymers, e.g., those described in DE-A03,942,643; buffers and salts to buffer the pH and ionic strength of the oral care compositions; and
- other optional ingredients that may be included are, e.g., bleaching agents such as peroxy compound, e.g., potassium peroxydiphosphate, effervescing systems such as sodium bicarbonate/citric acid systems, color change systems, and the like.

Such ingredients common in the art typically and collectively make-up less than 20% by weight of the oral care composition, and preferably, from 0.0 to 15% by weight, and most preferably, from about 0.01 to about 12% by weight of the oral care composition, including all ranges subsumed therein.

Suitable carrier humectants are preferably used in the oral care system of the present invention, in particular the activation system and they include, for example, glycerin, sorbitol, propylene glycol, dipropylene glycol, diglycerol, triacetin, mineral oil, polyethylene glycol (preferably, PEG-400), alkane diols like butane diol and hexanediol, ethanol, pentylene glycol, or a mixture thereof. The carrier humectants should, in any case, be substantially free of water, and preferably, anhydrous. The same, for example, can be used in solid form, whereby glycerin is the preferred carrier humectant. Such carriers are particularly suitable in compositions used in the second layer.

The carrier humectant is used to take the balance of the compositions up to 100%, and the same may be present in the range of from 10 to 90% by weight of the oral care composition. Preferably, the carrier humectant makes up from 25 to 80%, and most preferably, from 45 to 70% by weight of the oral care composition, based on total weight of the oral care composition and including all ranges subsumed therein.

The invention will now be illustrated by the following non-limiting Examples.

## Examples

Analysis method:

To measure gas evolution from a toothpaste the change in pressure in a sealed container was recorded. To conduct a measurement 350g of toothpaste was added to a clean dry bottle. To the lid of the bottle was mounted a short piece of tube (approx. 15cm) and a clamp. The bottle was placed into the appropriate climatic store (eg 40°C) for 24 hours with the tube open to the atmosphere to allow equilibration. Following acclimatisation, each bottle was sealed with the clamp. Following a pre-determined number of days at a specific temperature the pressure meter (Testo 510 - Differential Pressure Meter) was connected to the bottle tube, zeroed, the clamp released and the pressure within the container measured and recorded.

**Formulations:**

A series of formulations were prepared as follows(Examples A, 4, B and C are comparative examples):

| | Ex. A | Ex. 1 | Ex. 2 | Ex. 3 | Ex. 4 | Ex. B | Ex. C |
|---|---|---|---|---|---|---|---|
| Glycerol | | 62.04 | 62.04 | 62.04 | 62.04 | 62.04 | 62.04 |
| Sorbitol | 45.0 | | | | | | |
| Water | 27.11 | | | | | | |
| Sodium Lauryl Sulfate | 1.5 | 1.5 | 1.5 | 1.5 | 1.5 | 1.5 | 1.5 |
| Flavour | 1.0 | 1.1 | 1.1 | 1.1 | 1.1 | 1.1 | 1.1 |
| Thickening Silica | 8.0 | | | | | | |
| Abrasive Silica | 10.0 | | | | | | |
| Sodium Fluoride | 0.32 | | | | | | |
| Sodium carboxymethyl cellulose | 0.9 | | | | | | |
| Polyethylene glycol | 5.0 | | | | | | |
| Titanium dioxide | 1.0 | | | | | | |
| Sodium saccharin | 0.17 | 0.25 | 0.25 | 0.25 | 0.25 | 0.25 | 0.25 |
| Calcium Silicate | | 10.0 | 10.0 | 10.0 | 10.0 | 10.0 | 10.0 |
| Titanium Dioxide & Calcium Silicate | | 15.0 | 15.0 | 15.0 | 15.0 | 15.0 | 15.0 |
| Sodium monofluorophosphate | | 1.11 | 1.11 | 1.11 | 1.11 | 1.11 | 1.11 |
| Trisodium phosphate | | 2.5 | 3 | 3.2 | 3.35 | 3.75 | 5.0 |
| Monosodium Phosphate | | 2.5 | 2 | 1.8 | 1.65 | 1.25 | 0.0 |
| Calcium phosphate monobasic monohydrate | | 2.0 | 2.0 | 2.0 | 2.0 | 2.0 | 2.0 |
| Precipitated calcium carbonate | | 2.0 | 2.0 | 2.0 | 2.0 | 2.0 | 2.0 |

Example A was prepared by adding 80% of the water, sorbitol, polyethylene glycol, sodium saccharine, sodium fluoride and titanium dioxide to the mixer. The raw materials were mixed under vacuum for 10 minutes before adding thickening silica and sodium carboxymethyl cellulose and mixed for a further 10 minutes. Abrasive silica was added and mixed under vacuum for a further 10 minutes. Sodium lauryl sulfate was dissolved in the remaining water and added to the mixer. Finally, flavour was added the paste mixed under vacuum for a further 10 minutes.

Examples 1-4, B and C were prepared by adding glycerol, sodium lauryl sulfate and calcium silicate to a mixer. The mixture was heated and stirred under vacuum at 60°C for 30 minutes. Then titanium dioxide and calcium silicate, sodium monofluorophosphate and sodium saccharine were added, the mixture was stirred under vacuum at 60°C for 10 minutes. Then sodium phosphate, trisodium phosphate and precipitated calcium carbonate were added and the mixture stirred at 65°C for 30 minutes under vacuum. The temperature was reduced to 40°C and flavour was added, and the mixture stirred for 10 minutes. Finally, calcium phosphate was added, and the mixture stirred under vacuum for 5 minutes.

**Pressure Data:**

The paste samples were stored at 40°C and the clamp closed after 24 hours acclimatisation, after a further 72 hours the following pressure readings were recorded.

|  | Example A | Example 1 | Example 2 | Example 3 | Example | Example B | Example C |
|---|---|---|---|---|---|---|---|
| Pressure Change/Pa | 210 | 200 | 193 | 161 | 222 | 409 | 590 |
| Paste pH |  | 10.5 | 10.29 | 9.76 | 9.45 | 8.75 | 7.89 |

The data shows that examples 1-3 are similar to the example A control paste, examples 4,B and C with a pH of less than 9.5 are shown to gas on storage.

**Claims**

1. A non-aqueous personal care composition having a pH at 20°C of 9.5 or greater and comprising

    i) less than 1 wt% of the total composition of water;
    ii) and 0.1 to 10 wt% of the total composition of a calcium source comprising calcium carbonate,.

2. A personal care composition according to any preceding claim that further comprises a phosphate source.

3. A personal care composition according to claim 3 in which the phosphate source is a mixture of trisodium phosphate and sodium dihydrogen phosphate.

4. A personal care composition according to any preceding claim that further comprises calcium silicate.

5. A personal care composition according to any preceding claim in which the level of calcium carbonate is from 0.5 to 7 wt% of the total composition.

6. A personal care composition according to any preceding claim that is an oral care composition.

7. A personal care composition according to any preceding claim that is in the form of a toothpaste.

8. Use of a pH of greater than 9.5 to prevent gas formation in a toothpaste.

**Patentansprüche**

1. Nicht-wässrige Körperpflegezusammensetzung mit einem pH-Wert bei 20°C von 9,5 oder mehr und umfassend

    i) weniger als 1 Gew.-% der Gesamtzusammensetzung an Wasser;
    ii) und 0,1 bis 10 Gew.-% der Gesamtzusammensetzung einer Calciumquelle, die Calciumcarbonat umfasst.

**2.** Körperpflegezusammensetzung nach einem der vorhergehenden Ansprüche, die außerdem eine Phosphatquelle umfasst.

**3.** Körperpflegezusammensetzung nach Anspruch 3, in der die Phosphatquelle eine Mischung aus Trinatriumphosphat und Natriumdihydrogenphosphat ist.

**4.** Körperpflegezusammensetzung nach einem vorhergehenden Anspruch, die außerdem Calciumsilikat umfasst.

**5.** Körperpflegezusammensetzung nach einem vorhergehenden Anspruch, in der der Anteil des Calciumcarbonats 0,5 bis 7 Gew.-% der Gesamtzusammensetzung beträgt.

**6.** Körperpflegezusammensetzung nach einem vorhergehenden Anspruch, die eine Mundpflegezusammensetzung darstellt.

**7.** Körperpflegezusammensetzung nach einem vorhergehenden Anspruch, die in Form einer Zahnpasta vorliegt.

**8.** Verwendung eines pH-Werts von mehr als 9,5 zur Verhinderung von Gasbildung in einer Zahnpasta.

**Revendications**

**1.** Composition de soins personnels non aqueuse ayant un pH à 20°C de 9,5 ou plus et comprenant

    i) moins de 1 % en poids de la composition totale d'eau;
    ii) et 0,1 à 10 % en poids de la composition totale d'une source de calcium comprenant du carbonate de calcium.

**2.** Composition de soins personnels selon l'une quelconque des revendications précédentes, qui comprend en outre une source de phosphate.

**3.** Composition de soins personnels selon la revendication 3, dans laquelle la source de phosphate est un mélange de phosphate de trisodium et de dihydrogénophosphate de sodium.

**4.** Composition de soins personnels selon l'une quelconque des revendications précédentes, qui comprend en outre du silicate de calcium.

**5.** Composition de soins personnels selon l'une quelconque des revendications précédentes, dans laquelle le niveau de carbonate de calcium est de 0,5 à 7 % en poids de la composition totale.

**6.** Composition de soins personnels selon l'une quelconque des revendications précédentes, qui est une composition de soins buccodentaires.

**7.** Composition de soins personnels selon l'une quelconque des revendications précédentes, qui est sous la forme d'une pâte dentifrice.

**8.** Utilisation d'un pH supérieur à 9,5 pour empêcher la formation de gaz dans une pâte dentifrice.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 18106219 A
- US 5164172 A
- WO 2008015117 A
- WO 2012031786 A
- WO 2012031785 A
- DE 3942643 A0